# EUROPEAN PATENT APPLICATION

(11) **EP 4 707 265 A1**
(43) Date of publication of application: **11.03.2026**
(21) Application number: 24797211.0
(22) Date of filing: 26.04.2024
(51) Int. Cl.: C07C 17/367, C07C 19/08, C07C 21/18, C07C 21/185

(54) **METHOD FOR PRODUCING LOW-MOLECULAR-WEIGHT FLUORINE COMPOUND**

(30) Priority: 28.04.2023 JP 2023074905
(71) Applicant: Daikin Industries, Ltd., Osaka-shi, Osaka 530-0001 (JP); AGC INC., Chiyoda-ku, Tokyo 1008405 (JP)
(72) Inventor: FUKUSHIMA, Kazuaki, Osaka-shi, Osaka 559-0025 (JP); TAKAMOTO, Tamotsu, Osaka-shi, Osaka 559-0025 (JP); OGATA, Toshihiko, Osaka-shi, Osaka 559-0025 (JP); DEGUCHI, Yukari, Osaka-shi, Osaka 559-0025 (JP); TSUKAHARA, Yasunori, Osaka-shi, Osaka 559-0025 (JP); KISHIKAWA, Yosuke, Osaka-Shi, Osaka 530-0001 (JP); NAMIKAWA, Takashi, Osaka-Shi, Osaka 530-0001 (JP); MATSUI, Motoshi, Osaka-Shi, Osaka 530-0001 (JP); TAKAHIRA, Yusuke, Tokyo 100-8405 (JP); YAGISHITA, Masahito, Tokyo 100-8405 (JP)
(74) Representative: Müller-Boré & Partner Patentanwälte PartG mbB
(86) International application number: PCT/JP2024/016553
(87) International publication number: WO 2024/225469

(57) **Abstract**

The purpose is to improve the selectivity of a constituent monomer of a fluororesin among the low-molecular-weight fluorine compounds produced in a fluororesin decomposition method in which a temperature difference is provided so that the temperature of a product gas led out from a reaction vessel becomes lower than the temperature of a heated microwave absorber in the decomposition of the fluororesin. In a method for producing a low-molecular-weight fluorine compound by decomposing a fluororesin by bringing a heated microwave absorber into contact with a material containing a fluororesin in a reaction vessel provided with a carrier gas inlet and a decomposition gas outlet, (i) a temperature difference Delta 1(TR - TI), obtained by subtracting the carrier gas temperature TI (°C) from the temperature TR (°C) of the heated microwave absorber, is set to be more than 500°C and/or (ii) a temperature difference Delta 2(TR - TE), obtained by subtracting the product gas temperature TE (°C) at the outlet from the temperature TR (°C), is set to be 300°C or higher.

## Description

### Technical Field

The present invention relates to a technique for decomposing a fluororesin. More specifically, the present invention relates to a method for producing a low-molecular-weight fluorine compound by thermal decomposition of a fluororesin using microwave irradiation heating.

### Background Art

Fluororesins are widely used in coating materials for heat-resistant electric wires, semiconductor manufacturing materials, electronic components, and the like, utilizing excellent properties such as lubricity, heat resistance, and chemical resistance. In recent years, it has been actively studied to decompose and reuse a synthetic resin containing a fluororesin. For example, PTL 1 proposes a chemical recycling method for extracting, from a material containing PTFE, a tetrafluoroethylene low-molecular-weight monomer and a dimer thereof generated by thermally decomposing PTFE, the chemical recycling method including: a process of heating a fluidized bed reactor in which a fluidized bed formed of an inert particulate material is provided and into which the material has been supplied; and a process of introducing an inert gas as a fluidized gas into the fluidized bed reactor and extracting a tetrafluoroethylene low-molecular-weight monomer and a dimer thereof generated by heat decomposition of PTFE by the heating from the fluidized bed reactor together with the inert gas.

### Citation List

### Patent Literature

PTL 1: Japanese Patent Laid-open Publication No. 2004-346000

### Summary of Invention

### Technical Problem

The present inventors have focused on the fact that control of a temperature environment to which a low-molecular-weight fluorine compound such as a fluorine-based monomer generated from a fluororesin is exposed has not been studied in conventional chemical recycling techniques of fluororesins, and have found a method of controlling the temperature of a produced gas discharged from a reaction vessel so as to be intentionally lower than the temperature in a decomposition reaction system. However, as a result of further studies, in such a method, there is a problem in that there is room for improvement in the selectivity of a constituent monomer of a fluororesin among low-molecular-weight fluorine compounds to be produced.

Therefore, an object of the present invention is to improve the selectivity of a constituent monomer of a fluororesin among low-molecular-weight fluorine compounds to be produced in a fluororesin decomposition method in which a temperature difference (hereinafter, also referred to as "temperature difference Δ2(TR - TE)") is provided so that a temperature (hereinafter, also referred to as "temperature TE") of a produced gas discharged from a reaction vessel is controlled to be lower than a temperature in a decomposition reaction system, that is, a temperature (hereinafter, also referred to as "temperature TR") of a heated microwave absorber.

### Solution to Problem

As a result of intensive studies, the present inventors have found that by setting a temperature difference Δ1(TR - TI), obtained by subtracting a carrier gas temperature TI (°C) at a reaction vessel inlet from a temperature TR (°C) of a microwave absorber heated in a reaction vessel, to be higher than 500°C and/or setting a temperature difference Δ2(TR - TE), obtained by subtracting TE (°C) at a decomposed gas outlet of a produced gas discharged from a reaction vessel from a temperature TR (°C) of a microwave absorber heated in the reaction vessel, to be 300°C or higher, the selectivity of a constituent monomer of a fluororesin among low-molecular-weight fluorine compounds to be produced can be improved. The present invention has been completed based on these findings by conducting further studies.

That is, the present invention provides inventions of the following aspects.

Item 1. A method for producing a low-molecular-weight fluorine compound, the method including a decomposition step of heating a microwave absorber by irradiation with microwaves and bringing the heated microwave absorber into contact with a material containing a fluororesin to decompose the fluororesin into a low-molecular-weight fluorine compound in a reaction vessel provided with a carrier gas inlet and a decomposed gas outlet, in which
(i) a temperature difference Δ1(TR - TI), obtained by subtracting a carrier gas temperature TI (°C) at the inlet from a temperature TR (°C) of the heated microwave absorber, is set to be higher than 500°C.

Item 2. A method for producing a low-molecular-weight fluorine compound, the method including a decomposition step of heating a microwave absorber by irradiation with microwaves and bringing the heated microwave absorber into contact with a material containing a fluororesin to decompose the fluororesin into a low-molecular-weight fluorine compound in a reaction vessel provided with a carrier gas inlet and a decomposed gas outlet, in which
(ii) a temperature difference Δ2(TR - TE), obtained by subtracting a temperature TE (°C) of a produced gas discharged from the reaction vessel at the outlet from a temperature TR (°C) of the heated microwave absorber, is set to be 300°C or higher.

Item 3. The method according to item 1 or 2, in which an introduction rate R of a carrier gas from the inlet is 0.005 to 20 m/s.

Item 4. The method according to any one of items 1 to 3, in which a temperature TE (°C) of a produced gas discharged from the reaction vessel at the outlet is 20°C or higher and lower than 250°C.

Item 5. The method according to any one of items 1 to 4, in which
the microwave absorber is prepared in a form of a microwave absorber-containing layer that contains the microwave absorber and particles of the material containing a fluororesin and is provided so as to pass an airflow from the carrier gas inlet to the decomposed gas outlet, and
when an opening center of the outlet is designated as a point Pe and an intersection between a central axis of the microwave absorber-containing layer and a surface of the microwave absorber-containing layer on a side opposite to the outlet is designated as a point Pl, a ratio h/H of a height h of the microwave absorber-containing layer to a height H from the point Pl to the point Pe is 0.01 to 0.8.

Item 6. The method according to any one of items 1 to 5, in which a particle size d of the microwave absorber is 0.1 to 25 mm.

Item 7. The method according to item 5 or 6, in which the height h of the microwave absorber-containing layer is 0.1 to 200 mm.

Item 8. The method according to any one of items 2 to 7, in which the temperature difference Δ1(TR - TI), obtained by subtracting a carrier gas temperature TI (°C) at the inlet from a temperature TR (°C) of the heated microwave absorber, is set to be higher than 250°C.

### Advantageous Effects of Invention

According to the present invention, in a fluororesin decomposition method in which a temperature difference Δ2(TR - TE) is provided so that a temperature TE of a produced gas discharged from a reaction vessel is controlled to be lower than a temperature in a decomposition reaction system, that is, a temperature TR of a heated microwave absorber, the selectivity of a constituent monomer of a fluororesin among low-molecular-weight fluorine compounds to be produced can be improved.

### Brief Description of Drawings

[Fig. 1] Fig. 1 shows a schematic view of an example of a device (parallel up-flow type and tubular reaction vessel) used in a production method of the present invention.
[Fig. 2] Fig. 2 shows a schematic view of an example of a device (parallel down-flow type and tubular reaction vessel) used in the production method of the present invention.
[Fig. 3] Fig. 3 shows a schematic view of an example of a device (continuous tank type reaction vessel) used in the production method of the present invention.
[Fig. 4] Fig. 4 shows a schematic view of an example of a device (continuous tank type reaction vessel) used in the production method of the present invention. Description of Embodiments

A method for producing a low-molecular-weight fluorine compound of the present invention includes a decomposition step of heating a microwave absorber by irradiation with microwaves and bringing the heated microwave absorber into contact with a material containing a fluororesin to decompose the fluororesin into a low-molecular-weight fluorine compound in a reaction vessel provided with a carrier gas inlet and a decomposed gas outlet, in which the following settings (i) and/or (ii) are performed.
(i) A temperature difference Δ1(TR - TI), obtained by subtracting a carrier gas temperature TI (°C) at the inlet from a temperature TR (°C) of the heated microwave absorber is set to be higher than 500°C.
(ii) A temperature difference Δ2(TR - TE), obtained by subtracting a temperature TE (°C) of a produced gas discharged from the reaction vessel at the outlet from a temperature TR (°C) of the heated microwave absorber is set to be 300°C or higher.

Hereinafter, the method for producing a low-molecular-weight fluorine compound of the present invention will be described in detail.

### [1. Fluororesin]

In the present invention, a fluororesin is used as a raw material of a low-molecular-weight fluorine compound. The fluororesin is a fluorine compound having a molecular weight of 500 or more and a fluorine content (weight basis) of 57% or more.

Examples of the fluororesin include a fluorinated polyolefin and a fluorinated polyether.

The fluorinated polyolefin is a polymer or oligomer having a repeating unit derived from a fluorinated olefin.

Examples of the fluorinated olefin include tetrafluoroethylene (TFE), hexafluoropropylene (HFP), vinylidene fluoride (VDF), chlorotrifluoroethylene (CTFE), partially fluorinated or perfluorovinyl ether, and partially fluorinated or perfluoroallyl ether.

Examples of the perfluorovinyl ether include compounds represented by general formula CF₂=CF-O-R_{f} [wherein, R_{f} represents a perfluorinated linear, cyclic, or branched aliphatic group which may contain one or more oxygen atoms]. When R_{f} contains one or more oxygen atoms, examples of the R_{f} include groups represented by -(R^{a}_{f}O)ₙ(R^{b}_{f}O)ₘR^{c}_{f} [wherein, R^{a}_{f} and R^{b}_{f} are different linear or branched chain or cyclic perfluoroalkylene groups having 1 to 6 carbon atoms, particularly, 2 to 6 carbon atoms, m and n are independently integers of 0 to 10, and R^{c}_{f} is a perfluoroalkyl group having 1 to 6 carbon atoms]. When R_{f} contains no oxygen atoms, specific examples of the perfluorovinyl ether include perfluoromethyl vinyl ether (PMVE), perfluoropropyl vinyl ether, and perfluoroisopropyl vinyl ether.

Specific examples of the perfluorovinyl ether include perfluoro(methyl vinyl) ether (PMVE), perfluoro(ethyl vinyl) ether (PEVE), perfluoro(n-propyl vinyl) ether (PPVE-1), perfluoro-2-propoxypropyl vinyl ether (PPVE-2), perfluoro-3-methoxy-n-propyl vinyl ether, perfluoro-2-methoxy-ethyl vinyl ether, CF₃-(CF₂)₂-O-CF(CF₃)-CF₂-O-CF(CF₃)-CF₂-O-CF=CF₂, CF₂=CF-O-(CF₂)₃OCF₃, and CF₂=CFO(CF₂)₂OCF₃.

Examples of the perfluoroallyl ether include compounds represented by general formula CF₂=CF-CF₂-O-R_{f} [wherein, R_{f} is the same as R_{f} described above for the perfluorovinyl ether].

The fluorinated polyolefin may be a polymer or oligomer composed of a repeating unit derived from a fluorinated olefin, or may be a polymer or oligomer further containing a repeating unit derived from a non-fluorinated olefin in addition to a repeating unit derived from a fluorinated olefin. The non-fluorinated olefin is not particularly limited, and examples thereof include C₂ to C₈ olefins, more preferably include ethylene (E) and propylene (P). Among these fluorinated polyolefins, a polymer or oligomer composed of a repeating unit derived from a fluorinated olefin (that is, the molar ratio of the repeating unit derived from a non-fluorinated olefin in the fluorinated polyolefin is 0%) is preferable.

Suitable examples of the fluorinated polyolefin include homopolymers of TFE and the following copolymers: polymers containing repeating units derived from TFE and HFP; TFE, HFP, and VDF; TFE and CTFE; TFE, CTFE, and E or P; TFE and PMVE, PEVE, PPVE-1 or PPVE-2; TFE, E and HFP; and CTFE and E or P. Among these fluorinated polyolefins, homopolymers of TFE are preferable.

The fluorinated polyether is a polymer or oligomer having a fluorinated oxyalkylene repeating unit.

The fluorinated oxyalkylene repeating unit is represented by -C₁X₂₁O-, wherein 1 is an integer of 1 or more, X is hydrogen or fluorine and is linear or branched, and 1 and X may be different in each repetitive occurrence.

The fluororesin may be a fluorinated non-ionomer or a fluorinated ionomer (a fluoropolymer having a plurality of -SO₃⁻ and/or -COO⁻ groups as pendant or terminal groups). As the fluorinated ionomer, one used for the preparation of a membrane in a fuel cell or in an electrochemical reaction can be used without particular limitation.

The fluororesin may have a hydrogen atom or a functional group containing an atom other than fluorine at a terminal. The functional group containing an atom other than fluorine is not particularly limited, and examples thereof include an iodine group, a bromine group, a chlorine group, a hydroxyl group and an ester thereof, and a vinyl group.

The fluororesin may be used singly or in combination of a plurality of kinds thereof.

### [2. Material containing fluororesin]

The fluororesin is subjected to a decomposition step in the form of a material containing the fluororesin. The material may be made of only a fluororesin or may contain components other than the fluororesin. Examples of the other components include resin additives to be blended in a fluororesin composition, and more specific examples thereof include a filler, a dye, a lubricant, a curing agent, a curing accelerator, a thermal conductive agent, and an electrical conductive agent.

The properties of the material containing a fluororesin in the decomposition step are determined according to the conditions in the decomposition step, and are not particularly limited. When the material containing a fluororesin is a solid, the material is used in the form of particles. The particles are obtained by processing a fluororesin material into a particulate form by an arbitrary means. Examples of the means include a milling device and a pulverizing device.

The size of the particles is not particularly limited, and is, for example, 20 µm to 100 mm, preferably 100 µm to 10 mm, and more preferably 250 µm to 5 mm.

The material containing a fluororesin may be one obtained by a pretreatment that can be performed prior to the decomposition step. Such a pretreatment is not particularly limited as long as a material containing a fluororesin can be obtained, and examples thereof include a treatment of separating and removing a member not containing a fluororesin and/or a material other than a fluororesin from a material to be recycled, a treatment of removing oil and/or dust, and a fluorination treatment of a polymer material (a treatment of treating a polymer material with F₂ gas to convert a C-H bond into a C-F bond).

A method for supplying the material containing a fluororesin into the reaction vessel is not particularly limited, and may be appropriately selected by those skilled in the art. For example, when the material containing a fluororesin is particles, it is preferable to supply the particles into the reaction vessel by, for example, gravity feeding, and when the material containing a fluororesin is a liquid, the liquid can be supplied into the reaction vessel by, for example, spraying.

### [3. Reaction vessel]

As the reaction vessel, a vessel provided with a carrier gas inlet and a decomposed gas outlet for introducing and discharging a gas flow is used. Examples of the reaction vessel used in the present invention include a parallel flow type tubular reaction vessel and a continuous tank type reaction vessel.

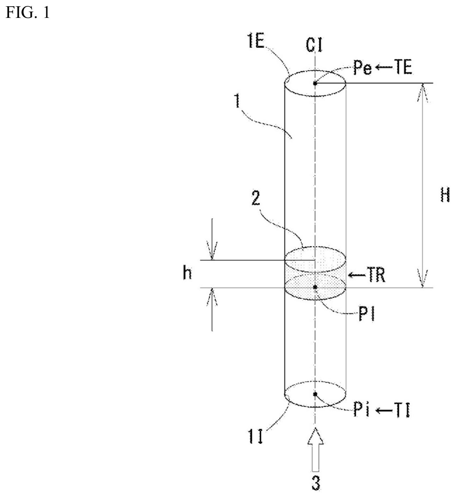

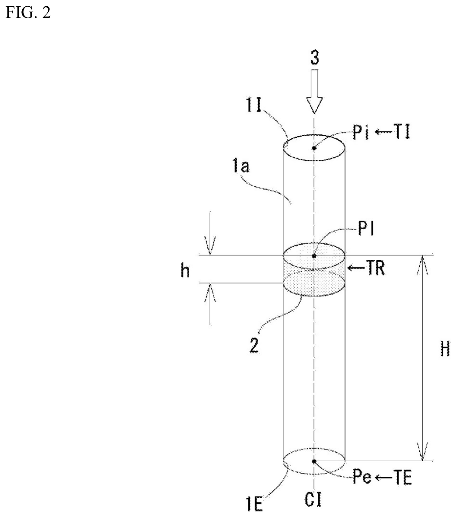

Specific examples of the parallel flow type tubular reaction vessel are shown in Figs. 1 and 2. A parallel flow type tubular reaction vessel 1 shown in Fig. 1 is provided with a carrier gas inlet 1I and a decomposed gas outlet 1E, and is configured such that the flow type of the gas flow of a carrier gas 3 is a parallel up-flow type. A parallel flow type tubular reaction vessel 1a shown in Fig. 2 is provided with a carrier gas inlet 1I and a decomposed gas outlet 1E, and is configured such that the flow type of the gas flow of a carrier gas 3 is a parallel down-flow type.

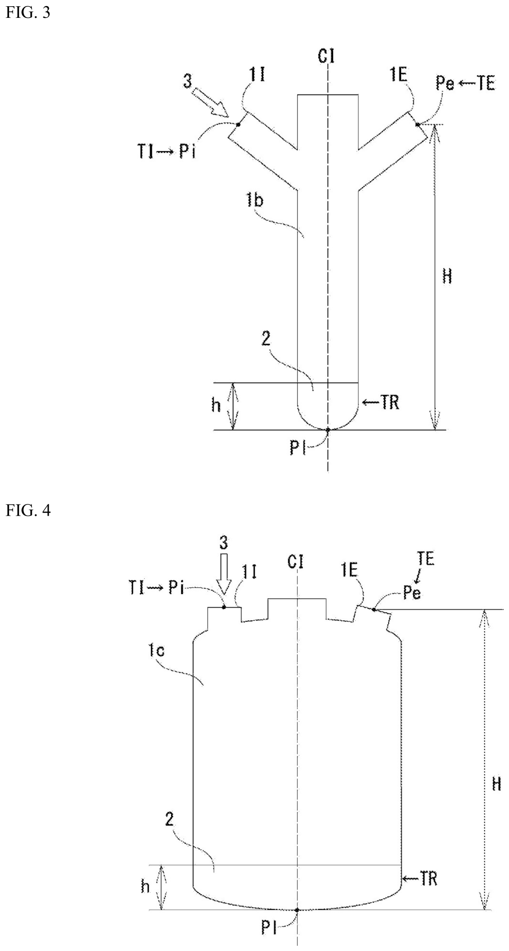

Specific examples of the continuous tank type reaction vessel are shown in Figs. 3 and 4. A continuous tank type reaction vessel 1b shown in Fig. 3 is provided with a carrier gas inlet 1I and a decomposed gas outlet 1E and is preferably used for a reaction at a small scale. A continuous tank type reaction vessel 1c shown in Fig. 4 is provided with a carrier gas inlet 1I and a decomposed gas outlet 1E and is preferably used for a reaction at a large scale. The continuous tank type reaction vessels 1b and 1c shown in Figs. 3 and 4 can include a stirring bar (not shown).

More specifically, the reaction vessel in the present invention can be incorporated into a device according to a general catalyst reactor, and in the device, a microwave absorber is used instead of a catalyst in the general catalyst reactor.

Since microwaves are used as the heating means in the present invention, the device may include a microwave generator. As the microwave generator, a known device can be appropriately selected, and examples of such a device include a diode, a magnetron, a gyrotron, a traveling wave tube, a klystron, a ubitron, and an amplitron.

### [4. Microwave absorber]

### [4-1. Examples of microwave absorber]

The microwave absorber is not particularly limited as long as it absorbs the irradiated microwave to generate heat at a temperature at which the fluororesin can be decomposed, and is in a solid state under the decomposition reaction conditions of the fluororesin and has chemically inert characteristics. The temperature TR of the microwave absorber heated in the decomposition step constitutes the temperature condition for advancing the decomposition reaction of the fluororesin and corresponds to the temperature in the reaction system of the decomposition reaction of the fluororesin.

Specific examples of the microwave absorber include metals, metal salts, intermetallic compounds, carbon, and carbides having the above characteristics. Preferably, a substance having a density of, for example, 1 to 10 g/cm³, preferably 1.5 to 7 g/cm³ can be used as the microwave absorber. Preferably, a substance having microwave absorbing ability with a dielectric loss of, for example, 0.01 to 30, preferably 0.1 to 11 can be used as the microwave absorber.

More specific examples of the microwave absorber include a single metal such as Ni, Pt, Co, or Pt, and an alloy such as Pt/Cu or Pt/Re. Examples of the metal salt include metal oxides, metal hydroxides, metal halides, and silicides. Examples of the intermetallic compound include molybdenum silicide and titanium boride. Examples of the carbon include graphite such as expanded graphite, activated carbon, carbon fiber, carbon nanotube, and carbon black. Examples of the carbide include silicon carbide, boron carbide, titanium carbide, and zirconium carbide. These microwave absorbers may be used singly or in combination of a plurality of kinds thereof. Among these microwave absorbers, carbides are preferable, and silicon carbide is more preferable.

### [4-2. Particle size d of microwave absorber]

In the present invention, the microwave absorber is preferably used in the form of particles. The particle size (hereinafter, also referred to as "particle size d") of the microwave absorber particles is not particularly limited, and is, for example, 0.1 to 25 mm. The particle size d when the mode of a microwave absorber-containing layer described later is a fixed bed is preferably 0.1 to 25 mm, more preferably 1 to 25 mm, still more preferably 2.8 to 25 mm, and further preferably 4.8 to 25 mm from the viewpoint of further increasing the temperature difference Δ2(TR - TE) and further improving the selectivity of the constituent monomer of the fluororesin, and the upper limit of the range of the particle size d may be 10 mm or less or 8 mm or less. The particle size d when the mode of a microwave absorber-containing layer described later is a fluidized bed is preferably 0.5 to 25 mm, more preferably 1 to 25 mm, still more preferably 2 to 10 mm, further preferably 2.8 to 25 mm, and even further preferably 4.8 to 25 mm from the viewpoint of further increasing the temperature difference Δ2(TR - TE) and further improving the selectivity of the constituent monomer of the fluororesin, and the upper limit of the range of the particle size d may be 15 mm or less, 10 mm or less, 5 mm or less, 2.5 mm or less, 2 mm or less, or 1.5 mm or less. Note that, in the present invention, the particle size d is an average of measurement values obtained by measuring, with a gauge, the maximum diameters in a fixed direction of randomly selected 20 particles. For the measurement with a gauge, visual observation, an optical microscope, or an electron microscope is used depending on the size of the particles.

Note that, in order to control the above (i), the upper limit of the particle size d of the microwave absorber can be set to be preferably 2.5 mm or less, more preferably 2 mm or less, and still more preferably 1.5 mm or less.

### [4-3. Mode of microwave absorber-containing layer]

A more specific mode of the microwave absorber during the decomposition reaction may be either a fixed bed or a fluidized bed.

The microwave absorber can be prepared in the reaction vessel in the mode of a microwave absorber-containing layer of a mixture containing the microwave absorber and particles of the material containing a fluororesin before the decomposition step.

The microwave absorber-containing layer is provided in the reaction vessel so as to allow an airflow from the carrier gas inlet to the decomposed gas outlet to pass therethrough. Specifically, in the parallel flow type tubular reaction vessels 1 and 1a shown in Figs. 1 and 2, the microwave absorber-containing layer is provided as a microwave absorber-containing layer 2 between the carrier gas inlet and the decomposed gas outlet, and in the continuous tank type reaction vessels 1b and 1c shown in Figs. 3 and 4, the microwave absorber-containing layer is provided as a microwave absorber-containing layer 2 at the bottom of the reaction vessel.

The used amount of the microwave absorber with respect to the amount of the particles of the material containing a fluororesin is not particularly limited, and is, for example, 0.1 to 1000 parts by weight, preferably 1 to 100 parts by weight, more preferably 10 to 50 parts by weight, and still more preferably 15 to 45 parts by weight with respect to 1 part by weight of the particles of the material containing a fluororesin.

In the initial stage (before the start of the decomposition step), the microwave absorber-containing layer 2 has a height h when the contact surface with the gas phase is leveled. The height h refers to the height of the microwave absorber-containing layer 2 at a central axis CI (in the forms of Figs. 1 to 4, the central axis CI of the microwave absorber-containing layer 2 coincides with the central axes of the reaction vessels 1, 1a, 1b, and 1c).

Specific examples of the height h of the microwave absorber-containing layer 2 include 0.1 to 200 mm, and from the viewpoint of further increasing the temperature difference Δ2(TR - TE) and further improving the selectivity of the constituent monomer of the fluororesin, the height h is preferably 1 to 100 mm, more preferably 3 to 50 mm, and still more preferably 5 to 30 mm. The height h when the mode of the microwave absorber-containing layer 2 is a fixed bed is preferably 3 to 50 mm and more preferably 5 to 30 mm, and the height h when the mode thereof is a fluidized bed is preferably 5 to 30 mm.

When an opening center of the outlet 1E of the reaction vessels 1, 1a, 1b, and 1c is designated as a point Pe and an intersection between the central axis CI of the microwave absorber-containing layer 2 and the outlet 1E of the microwave absorber-containing layer 2 is designated as a point Pl, the ratio (h/H) of the height h of the microwave absorber-containing layer 2 when the height H from the point P1 to the point Pe is taken as 1 is, for example, 0.01 to 0.8, and from the viewpoint of further increasing the temperature difference Δ2(TR - TE) and further improving the selectivity of the constituent monomer of the fluororesin, the ratio is preferably 0.01 to 0.5, more preferably 0.05 to 0.4, still more preferably 0.1 to 0.3, and further preferably 0.15 to 0.25.

### [5. Carrier gas]

### [5-1. Examples of carrier gas]

The carrier gas introduced from the carrier gas inlet of the reaction vessel is not particularly limited as long as it has chemically inert characteristics under the decomposition reaction conditions of the fluororesin, and examples thereof include water vapor, nitrogen, and a rare gas (such as Xe, Ar, Ne, or He). These carrier gases may be used singly or in combination of a plurality of kinds thereof. Among these carrier gases, water vapor and nitrogen are preferable.

### [5-2. Temperature TI of carrier gas at inlet and setting thereof]

In the present invention, the temperature TI of the carrier gas at the inlet is a temperature at the opening center (hereinafter, also referred to as "point Pi") of the inlet 1I.

In the present invention, in order to improve the selectivity of the constituent monomer of the fluororesin among low-molecular-weight fluorine compounds to be produced, the temperature TI of the carrier gas at the inlet can be set so as to achieve the temperature difference Δ1(TR - TI) shown in the above (i).

The temperature difference Δ1(TR - TI) is not particularly limited as long as it is higher than 500°C, and from the viewpoint of further improving the selectivity of the constituent monomer of the fluororesin, the temperature difference Δ1(TR - TI) is preferably 530°C or higher, 540°C or higher, 550°C or higher, 570°C or higher, 590°C or higher, or 600°C or higher, more preferably 620°C or higher, 640°C or higher, 660°C or higher, 680°C or higher, 700°C or higher, or 720°C or higher, and still more preferably 740°C or higher, 760°C or higher, or 780°C or higher. The upper limit of the temperature difference Δ1(TR - TI) is also not particularly limited, and is, for example, 1000°C or lower, 900°C or lower, or 800°C or lower.

Note that, in the present invention, when the setting of (ii) is performed, the temperature difference Δ1(TR - TI) is allowed to be higher than 250°C (preferably 300°C or higher, more preferably 350°C or higher, still more preferably 400°C or higher, and further preferably 450°C or higher).

In the present invention, the value obtained by dividing the temperature difference Δ1(TR - TI) by the temperature TR (°C) is, for example, 0.5 or more, preferably 0.85 or more, more preferably 0.9 or more, still more preferably 0.93 or more, further preferably 0.94 or more, and even further preferably more than 0.97, and from the viewpoint of further improving the selectivity of the constituent monomer of the fluororesin, the value is preferably 0.965 or more, more preferably 0.972 or more, and still more preferably 0.976 or more. The upper limit of the value obtained by dividing the temperature difference Δ1(TR - TI) by the temperature TR is also not particularly limited, and is, for example, 0.990 or less, 0.985 or less, or 0.980 or less.

Note that, in order to control the above (i), the value obtained by dividing the temperature difference Δ1(TR - TI) by the temperature TR (°C) can be set to be preferably more than 0.97, more preferably 0.965 or more, still more preferably 0.972 or more, and further preferably 0.976 or more, and in order to control Δ1(TR - TI) to be larger, the value can be set to be higher.

In order to control the above (ii), the value obtained by dividing the temperature difference Δ1(TR - TI) by the temperature TR (°C) can be set to be, for example, 0.5 or more, preferably 0.85 or more, more preferably 0.9 or more, still more preferably 0.93 or more, and further preferably 0.94 or more, and can also be controlled to be more than 0.97, more preferably 0.965 or more, still more preferably 0.972 or more, and further preferably 0.976 or more. The value can be set to be higher in order to control Δ2(TR - TE) to be larger.

In the present invention, the carrier gas at the inlet may be preliminarily heated or may not be preliminarily heated. That is, the present invention may or may not include a step of preliminarily heating the carrier gas. Specific examples of the temperature TI include 10 to 400°C, preferably 15 to 300°C, and more preferably 20 to 200°C. More specifically, as a preferable example in the case of not preliminarily heating, the temperature is, for example, 10 to 30°C, preferably 13 to 25°C, and still more preferably 15 to 23°C, and as a preferable example in the case of preliminarily heating, the temperature is, for example, 80 to 400°C, preferably 90 to 200°C, and more preferably 100 to 150°C.

### [5-3. Introduction rate R of carrier gas]

The introduction rate (hereinafter, also referred to as "introduction rate R") of the carrier gas from the carrier gas inlet is not particularly limited, and is, for example, 0.005 to 20 m/s, and from the viewpoint of further increasing the temperature difference Δ2(TR - TE) and further improving the selectivity of the constituent monomer of the fluororesin, the introduction rate is preferably 0.007 to 2 m/s, more preferably 0.009 to 0.2 m/s, still more preferably 0.011 to 0.08 m/s, and further preferably 0.013 to 0.02 m/s.

### [6. Decomposition reaction]

In the decomposition reaction, the microwave absorber is heated by irradiation with microwaves in the reaction vessel, and the heated microwave absorber is brought into contact with particles of a material containing a fluororesin to decompose the fluororesin into a low-molecular-weight fluorine compound.

### [6-1. Reaction temperature TR]

The temperature TR of the heated microwave absorber may be a temperature at which the fluororesin is decomposed. Specific examples of the temperature TR include 280 to 1500°C, preferably 490 to 1400°C or 530 to 1300°C, more preferably 640 to 1100°C, still more preferably 680 to 1000°C, and further preferably 760 to 900°C. The microwave irradiation conditions (wavelength and frequency) for heating the microwave absorber may be appropriately set according to the temperature TR.

Note that, in order to control the above (i), the temperature TR of the heated microwave absorber can be set to be preferably 490 to 1400°C or 530 to 1300°C, more preferably 640 to 1100°C, still more preferably 680 to 1000°C, and further preferably 760 to 900°C, and in order to control Δ1(TR - TI) to be larger, the temperature TR can be set to be higher.

The temperature TR is a temperature obtained by measuring a temperature at a central position in the height h direction of the microwave absorber-containing layer from the lateral surface side of the reaction vessel in a state where the reaction vessel is thermally insulated. The heat insulation of the reaction vessel at the time of measuring the temperature TR is performed by surrounding the reaction vessel with ceramic wool so as to have a thickness of 20% of the maximum outer peripheral width of the reaction vessel. For the temperature measurement, an optical fiber thermometer or a non-contact thermometer can be used. Note that the heat insulation method constitutes the measurement conditions of the temperature TR specified in the present invention, and the production method of the present invention is not limited to the mode in which this heat insulation method is performed.

### [6-2. Temperature TE of produced gas at outlet and setting thereof]

The low-molecular-weight fluorine compound to be produced by the decomposition reaction of the fluororesin is discharged as a gas (produced gas) from the outlet 1E to the outside of the reaction vessel. In the present invention, the temperature TE of the produced gas at the outlet is a temperature at the opening center (hereinafter, also referred to as "point Pe") of the outlet 1E. The temperature TE in the present invention is a temperature at the time when the decomposed gas (produced gas) having passed through the microwave absorber-containing layer reaches the point Pe of the decomposed gas outlet of the reaction vessel without being cooled by a rapid cooling method. Here, the rapid cooling method refers to a method of cooling the decomposed gas so as to have a temperature lower than a temperature in the case of natural cooling (that is, a temperature which the decomposed gas having passed through the microwave absorber-containing layer exhibits at a time point when the decomposed gas is naturally cooled before reaching the decomposed gas outlet of the reaction vessel and the decomposed gas reaches the decomposed gas outlet), specific methods are known in the art, and examples thereof include a method of expanding the decomposed gas (produced gas), a method of cooling the decomposed gas using a separately prepared cooling gas, and a method of cooling the decomposed gas using a cooling liquid.

In the present invention, in order to improve the selectivity of the constituent monomer of the fluororesin among low-molecular-weight fluorine compounds to be produced, the temperature TE of the produced gas at the outlet can be set so as to achieve the temperature difference Δ2(TR - TE) shown in the above (ii).

The temperature difference Δ2(TR - TE) is not particularly limited as long as it is 300°C or higher, and from the viewpoint of further improving the selectivity of the constituent monomer of the fluororesin, the temperature difference Δ2(TR - TE) is preferably 400°C or higher, more preferably 460°C or higher, 480°C or higher, 500°C or higher, 520°C or higher, 540°C or higher, 560°C or higher, 580°C or higher, or 600°C or higher, still more preferably 610°C or higher, 630°C or higher, 650°C or higher, 670°C or higher, 690°C or higher, or 710°C or higher, and further preferably 730°C or higher, 750°C or higher, or 770°C or higher. The upper limit of the temperature difference Δ2(TR - TE) is also not particularly limited, and is, for example, 1000°C or lower, 900°C or lower, or 850°C or lower.

Note that, in order to control the above (i), Δ2(TR - TE) can be set to be preferably 460°C or higher, 480°C or higher, 500°C or higher, 520°C or higher, 540°C or higher, 560°C or higher, 580°C or higher, or 600°C or higher, more preferably 610°C or higher, 630°C or higher, 650°C or higher, 670°C or higher, 690°C or higher, or 710°C or higher, and still more preferably 730°C or higher, 750°C or higher, or 770°C or higher. Δ2(TR - TE) can be set to be larger in order to control Δ1(TR - TI) to be larger.

Specific examples of the temperature TE include 20°C or higher and lower than 250°C. The upper limit of the temperature range (20°C or higher and lower than 250°C) is preferably 200°C or lower, more preferably 150°C or lower, still more preferably 100°C or lower, and further preferably 90°C or lower, 85°C or lower, or 80°C or lower, and the lower limit thereof is also 30°C or higher, 40°C or higher, 50°C or higher, 55°C or higher, 60°C or higher, 65°C or higher, 70°C or higher, or 75°C or higher.

Note that, in order to control the above (i), the temperature TE can be set to be preferably 50°C or higher, 55°C or higher, 60°C or higher, 65°C or higher, 70°C or higher, or 75°C or higher, and in order to control Δ1(TR - TI) to be larger, the temperature TE can be set to be higher.

### [6-3. Decomposition product]

The low-molecular-weight fluorine compound which is a product of the decomposition reaction is a compound having a lower molecular weight than the fluororesin generated from the fluororesin which is a raw material, and usually includes a plurality of kinds of compounds. According to the present invention, the selectivity of the constituent monomer of the fluororesin among low-molecular-weight fluorine compounds to be produced is high (that is, the proportion of the constituent monomer of the fluororesin to the low-molecular-weight fluorine compound to be produced is high).

Among the low-molecular-weight fluorine compounds to be produced, the constituent monomer of the target fluororesin is determined according to the polymer configuration of the fluororesin as a raw material. Specific examples of the constituent monomer of the fluororesin include compounds represented by general formula CF₂=CF-R^{d}_{f} [wherein, R^{d}_{f} represents F or a perfluoroalkyl group having 1 to 10, preferably 1 to 5 carbon atoms], and preferably include tetrafluoroethylene (TFE) and hexafluoropropylene (HFP). Other specific examples of the fluoroolefin include vinylidene fluoride (VDF), chlorotrifluoroethylene (CTFE), perfluorovinyl ether, and perfluoroallyl ether.

Examples of the compound other than the constituent monomer of the fluororesin among the low-molecular-weight fluorine compounds to be produced include a polymer having a low degree of polymerization in which the decomposition has not progressed to the constituent monomer of the fluororesin (a dimer, a trimer, or an oligomer having a higher degree of polymerization, which is gaseous at the temperature TE), a by-product generated by further causing a side reaction of the constituent monomer of the fluororesin (for example, octafluorocyclobutane or the like), and other by-products.

The low-molecular-weight fluorine compound (produced gas), which is a product, is discharged from the outlet of the reaction vessel by the airflow and is recovered. As the recovery method, the produced gas may be appropriately cooled and/or concentrated.

During the decomposition step, additional supply of the material containing a fluororesin may not be performed (that is, the process may be a batch process), or the fluororesin may be additionally supplied continuously or intermittently.

### Examples

Hereinafter, the present invention will be more specifically described by means of Examples; however, the present invention is not limited to the following Examples.

The reaction vessel 1b of the continuous tank type reaction vessel shown in Fig. 3 was prepared. In the cylindrical reaction vessel 1b, the microwave absorber-containing layer 2 containing particles of the material containing a microwave absorber having a predetermined particle size d and a fluororesin (PTFE, that is, a homopolymer of tetrafluoroethylene) was provided at a predetermined height h (cm). The size of the particles of the material containing a fluororesin was 20 µm, and the used amount of the microwave absorber was 20 to 40 parts by weight with respect to 1 part by weight of the particles of the material containing a fluororesin.

The carrier gas (nitrogen) 3 was introduced from the inlet 1I at a predetermined carrier gas temperature TI and a predetermined introduction rate R, the microwave absorber in the microwave absorber-containing layer 2 was heated to a predetermined temperature TR by a microwave generator (not shown) while being stirred with a stirring bar (not shown), and the temperature TE of the generated gas at the outlet 1E (specifically, the point Pe that is the opening center of the outlet 1E) was measured. Note that the temperature TR was measured by surrounding the outer periphery of the reaction vessel with ceramic wool so as to have a thickness of 20% of the vessel outer diameter to insulate the reaction vessel, and measuring the temperature at the central position in the height h direction of the microwave absorber-containing layer 2 from the lateral surface side of the reaction vessel using a non-contact thermometer. The produced gas was recovered from the outlet 1E, and the mass fraction of main three components was derived from the composition analysis result. Various set conditions and composition analysis results of the produced gas are shown in Table 1.

**[Table 1]**

| | Example | | |
|---|---|---|---|
| | 1 | 2 | 3 |
| Carrier gas temperature TI (°C) | 18.6 | 18.4 | 18.1 |
| Microwave absorber temperature TR (°C) | 800 | 700 | 550 |
| Microwave absorber | SiC | | |
| Microwave absorber particle size d (mm) | 1 | | |
| Vessel inner diameter (mm) | 12 | | |
| Height of point P1 - point Pe H (mm) | 75 | | |
| Height h (mm) | 15 | | |
| h/H | 0.2 | | |
| Carrier gas introduction rate R (m/s) | 0.015 | | |
| Outlet temperature TE (°C) | 76.8 | 65.5 | 52.8 |
| Δ1(TR - TI)/TR | 0.977 | 0.974 | 0.967 |
| Δ1(TR - TI) (°C) | 781.4 | 681.6 | 531.9 |
| A2(TR - TE) (°C) | 776 | 675 | 528 |
| **TFE (Tetrafluoroethylene)** | **94.7%** | **94.2%** | **91.3%** |
| HFP (Hexafluoropropylene) | 5.0% | 5.4% | 4.3% |
| C318 (Octafluorocyclobutane) | 0.3% | 0.5% | 4.4% |

As shown in Table 1, according to Examples 1 to 3, it was found that among the low-molecular-weight fluorine compound to be produced, the ratio of by-products (HFP and C318) was extremely low, and the selectivity of the constituent monomer (TFE) of the fluororesin (homopolymer of TFE) was extremely high. Note that the constituent components of the produced gas were TFE, HFP, and C138, and other products, and in all of Examples 1 to 3, it was also confirmed that TFE, HFP, and C138 accounted for about 95% or more of the area value of the gas chromatograph (detector FID: Flame Ionization Detector) among the constituent components, and the proportions of the remaining other products to the produced gas were equivalent between Examples 1 to 3.

### Reference Signs List

1, 1a, 1b, 1c Reaction vessel
1I Carrier gas inlet
TI Carrier gas temperature at inlet 1I
Pi Opening center of inlet 1I
1E Carrier gas outlet
TE Produced gas temperature at outlet 1E
Pe Opening center of outlet 1E
2 Microwave absorber-containing layer
CI Central axis of microwave absorber-containing layer 2
Pl Intersection between central axis CI and surface of microwave absorber-containing layer 2 on side opposite to outlet 1E
TR Temperature of heated microwave absorber
3 Carrier gas
H Height from point Pl to point Pe
h Height of microwave absorber-containing layer 2

## Claims

1. A method for producing a low-molecular-weight fluorine compound, the method comprising a decomposition step of heating a microwave absorber by irradiation with microwaves and bringing the heated microwave absorber into contact with a material containing a fluororesin to decompose the fluororesin into a low-molecular-weight fluorine compound in a reaction vessel provided with a carrier gas inlet and a decomposed gas outlet, wherein
(i) a temperature difference Δ1(TR - TI), obtained by subtracting a carrier gas temperature TI (°C) at the inlet from a temperature TR (°C) of the heated microwave absorber, is set to be higher than 500°C.

2. A method for producing a low-molecular-weight fluorine compound, the method comprising a decomposition step of heating a microwave absorber by irradiation with microwaves and bringing the heated microwave absorber into contact with a material containing a fluororesin to decompose the fluororesin into a low-molecular-weight fluorine compound in a reaction vessel provided with a carrier gas inlet and a decomposed gas outlet, wherein
(ii) a temperature difference Δ2(TR - TE), obtained by subtracting a temperature TE (°C) of a produced gas discharged from the reaction vessel at the outlet from a temperature TR (°C) of the heated microwave absorber, is set to be 300°C or higher.

3. The method according to claim 1 or 2, wherein an introduction rate R of a carrier gas from the inlet is 0.005 to 20 m/s.

4. The method according to claim 1 or 2, wherein a temperature TE (°C) of a produced gas discharged from the reaction vessel at the outlet is 20°C or higher and lower than 250°C.

5. The method according to claim 1 or 2, wherein
the microwave absorber is prepared in a form of a microwave absorber-containing layer that contains the microwave absorber and particles of the material containing a fluororesin and is provided so as to pass an airflow from the carrier gas inlet to the decomposed gas outlet, and
when an opening center of the outlet is designated as a point Pe and an intersection between a central axis of the microwave absorber-containing layer and a surface of the microwave absorber-containing layer on a side opposite to the outlet is designated as a point Pl, a ratio h/H of a height h of the microwave absorber-containing layer to a height H from the point Pl to the point Pe is 0.01 to 0.8.

6. The method according to claim 1 or 2, wherein a particle size d of the microwave absorber is 0.1 to 25 mm.

7. The method according to claim 5, wherein the height h of the microwave absorber-containing layer is 0.1 to 200 mm.

8. The method according to claim 2, wherein the temperature difference Δ1(TR - TI), obtained by subtracting a carrier gas temperature TI (°C) at the inlet from a temperature TR (°C) of the heated microwave absorber, is set to be higher than 250°C.
